# EUROPÄISCHE PATENTSCHRIFT

(11) **EP 0 342 564 B1**
(45) Veröffentlichungstag und Bekanntmachung des Hinweises auf die Patenterteilung: **02.02.1994**
(21) Anmeldenummer: 89108662.1
(22) Anmeldetag: 13.05.1989
(51) Int. Cl.: C12N 7/02, G01N 33/569

(54) **Verfahren zur Gewinnung eines Testantigens für die Diagnose der Bovinen Herpesvirus Typ 1 (BHV 1)-Infektion**
Method for the production of a test antigen for the diagnosis of bovine herpes virus type 1 (BHV 1) infection
Procédé de production d'un antigène d'essai pour le diagnostic d'une infection de virus herpés bovin de type 1 (BHV 1)

(30) Priorität: 18.05.1988 DE 3816885
(43) Veröffentlichungstag der Anmeldung: 23.11.1989
(73) Patentinhaber: BEHRINGWERKE Aktiengesellschaft, 35001 Marburg (DE)
(72) Erfinder: Bengelsdorff, Hans-Joachim, Dr., D-3550 Marburg (DE); Koschel, Eckhard, D-3578 Schwalmstadt (DE)

(56) Entgegenhaltungen:
- DE-A- 2 033 946
- JOURNAL OF BIOLOGICAL STANDARDIZATION, Band 15, Nr. 3, 1987, Seiten 213-222,The International Association of Biological Standardization, London, GB; C. LEQ. DARCEL et al.: "ELISA for bovine herpesvirus 1 (BHV1) antibody with HIRTsupernatant"
- VACCINE, Band 5, September 1987, Seiten 239-243, Butterworth& Co. Ltd, Guidlford, GB; M. TRUDEL et al.: "Vaccinationof rabbits with a bovine herpesvirus type 1 subunit vaccine: adjuvant effect ofISCOMs"

## Beschreibung

Die Erfindung betrifft ein Verfahren zur Gewinnung eines intrakutan anzuwendenden Testantigens zum Nachweis der BHV 1-Infektion beim Rind.

Die Infektion von Rindern mit dem BHV 1 führt klinisch zu einer Erkrankung der Atemwege (Infektiöse Bovine Rhinotracheitis = IBR) oder zu Erkrankungen an den Geschlechtsorganen (Infektiöse Pustulöse Vulvovaginitis = IPV beim weiblichen bzw. Infektiöse Balanoposthitis = IBP beim männlichen Tier) Nach Abklingen der akuten Krankheitssymptome geht die BHV 1-Infektion in eine latente Phase über, die mehr oder weniger lebenslang bestehen bleibt, doch können Streßsituationen zu Reaktivierungen der latenten BHV 1-Infektion führen.

Die klinische Diagnose einer BHV 1-Infektion wird virologisch durch Isolierung des Erregers, am häufigsten aber serologisch durch Nachweis der spezifischen Antikörper gestellt. Der serologische Antikörpernachweis wird derzeit geführt durch den Serumneutralisationstest oder durch den ELISA (Enzyme Linked Immuno-Sorbent Assay). Beiden serologischen Testen ist gemeinsam, daß eine Blutprobe dem betreffenden Tier entnommen und in einem geeigneten Institut untersucht werden muß. Eine direkt am Tier vorzunehmende Testmethode mit Hilfe eines intrakutan zu injizierenden Testantigens wird zwar von der Praxis dringend gewünscht, doch steht ein hinreichend spezifisch wirkendes Testantigen bislang nicht zur Verfügung.

Testantigene sind schon hergestellt worden aus ungereinigten oder gereinigten, inaktivierten BHV 1-Suspensionen, die von bovinen Zellkulturen gewonnen worden sind. Solchen Testantigenen wurden auch schon Immunstimulantien, wie beispielsweise Saponin, Extrakt aus Quillaja saponaria oder Detergentien, beispielsweise ^{R}Tween 80, zugesetzt, ohne daß dadurch der Zweck, eine ausreichend sichere und spezifisch wirksame Nachweisreaktion einer BHV 1-Infektion beim Rind, erreicht werden konnte.

Es wurde nun ein Verfahren gefunden, das es gestattet, durch Kombination von Konzentrierungs- und Reinigungsmethoden aus BHV 1-Kulturvirussuspensionen nach Inaktivierung des Viruskonzentrates ein Testantigen herzustellen, das nach intrakutaner Injektion bei Rindern durch Messung der Hautdickenzunahme eindeutig erkennen läßt, ob die Tiere einer BHV 1-Infektion ausgesetzt waren oder nicht.

Ausgangspunkt für dieses Verfahren ist eine in bovinen Zellkulturen vermehrte und nach üblicher Vorreinigung durch Zentrifugation und Klärfiltration gewonnene BHV 1-Kulturvirussuspension.

Das Verfahren ist dadurch gekennzeichnet, daß diese BHV 1-Kulturvirussuspension durch Ultrafiltration konzentriert, durch nachfolgende Waschung des Viruskonzentrates mit einer isotonischen gepufferten, vorzugsweise phosphatgepufferten Salzlösung von niedermolekularen, löslichen Begleitsubstanzen befreit, durch anschließende Viruspräzipitation mittels Polyäthylenglykol, Mol.-Gew. 6000, gereinigt und konzentriert sowie in isotonischer gepufferter, vorzugsweise phosphatgepufferter Salzlösung gelöst wird. Danach wird das hoch gereinigte und konzentrierte Testantigen mit einem Virusinaktivierungsmittel inaktiviert.

Gegenstand der Erfindung ist deshalb die Herstellung eines intrakutan beim Rind anzuwendenden BHV-1-Testantigens, das aus einer durch Zentrifugation und Klärfiltration vorgereinigten BHV 1-Kulturvirussuspension durch eine Verfahrensfolge gewonnen wird, die gekennzeichnet ist durch Viruskonzentrierung und -reinigung, vorzugsweise mittels Ultrafiltration, nachfolgender Waschung des Viruskonzentrates mit isotonischer gepufferter, vorzugsweise phosphatgepufferter Salzlösung, Viruspräzipitation, vorzugsweise mittels Polyäthylenglykol, vorzugsweise mit Molekulargewicht 6000, und suspendieren des Viruspräzipitates in isotonischer gepufferter, vorzugsweise phosphatgepufferter Salzlösung. Das so gewonnene Testantigen wird anschließend mit einem Virusinaktivierungsmittel in an sich bekannter Weise inaktiviert und stellt dann das anwendungsbereite BHV 1-Testantigen dar.

Gegenstand der Erfindung ist besonders ein Verfahren zur Gewinnung eines Bovinen Herpes Virus Typ 1 (BHV-1) Antigens aus einer zellfrei filtrierten Suspension von BHV 1-Virus, worin die Virussuspension konzentriert und Verunreinigungen abgetrennt, das Viruskonzentrat mit einer Pufferlösung gewaschen, die Viren ausgefällt, das Viruspräzipitat in einer Pufferlösung suspendiert und die Viren inaktiviert werden, dadurch gekennzeichnet, daß Verunreinigungen abgetrennt werden, die ein Filter mit der Porenweite 0,06 -0,08 µm passieren.

Zweckmäßigerweise gewinnt man das BHV 1-Testantigen in der Weise, daß in bovinen Zellkulturen, besonders primären Organkulturen wie solchen aus Nieren, Lungen oder Hoden oder auf permanenten bovinen Zellinien vermehrte BHV 1-Kulturvirussuspension, vorzugsweise durch Zentrifugation und Klärfiltration über Membranfilter von Zellresten befreit wird. Die filtrierte Kulturvirussuspension wird, vorzugsweise durch Ultrafiltration über Filterschichten mit Porenweiten von 0,06 bis 0,08 µm, vorzugsweise von 0,07 µm, 5- bis 15-fach, vorzugsweise 10-fach konzentriert. Danach wird das Viruskonzentrat mit dem 3- bis 10-fachen, vorzugsweise 5-fachen Volumen einer auf einem pH-Wert von 6,9 - 7,5, vorzugsweise 7,2 eingestellten, isotonischen gepufferten, vorzugsweise phosphatgepufferten Salzlösung (PBS) gewaschen. Durch diesen Verfahrensschritt werden zahlreiche niedermolekulare Substanzen aus dem Kulturviruskonzentrat entfernt, welche die Spezifität des Testantigens beim Tier beeinträchtigen. Die Waschflüssigkeit wird durch Ultrafiltration dem Viruskonzentrat wieder entzogen.

Die Virusreinigung und -konzentrierung wird vorzugsweise durch eine Viruspräzipitation, vorzugsweise mit 5 bis 20, vorzugsweise 10 Gew.% eines Polyäthylenglykols, vorzugsweise mit einem Mol.-Gew. von 6000 (PEG 6000) fortgesetzt. Nach 5- bis 24-stündigem, vorzugsweise 18-stündigem Rühren bei 2 bis 12° C, vorzugsweise 5 bis 8° C, wird das Viruspräzipitat durch Zentrifugation, vorzugsweise in einer Kühlzentrifuge gewonnen. Die Rücklösung des Viruspräzipitates erfolgt vorzugsweise mit PBS und zwar in einem Volumen von 1 bis 10, vorzugsweise 5% der ursprünglichen BHV 1-Kulturvirussuspension.

Das vorzugsweise 20-fach angereicherte, gereinigte BHV 1-Antigen wird nun in bekannter Weise inaktiviert, wozu es mit 0,025 bis 0,1 % eines Inaktivierungsmittels, vorzugsweise Äthylenimin versetzt und bei einer Temperatur von 4 bis 40°C, vorzugsweise 26°C 12 bis 60 Stunden, vorzugsweise 48 Stunden gerührt wird. Gegebenenfalls werden Restmengen des Inaktivierungsmittels in dem BHV 1-Testantigen abgebunden.

Ein nach dem beschriebenen Verfahren hergestelltes BHV 1-Testantigen enthält vor der Inaktivierung mindestens 10^{8,5}, maximal 10⁹ GKID₅₀ (Gewebekultur infektiöse Dosis 50 %).

### Beispiel 1

60 gerollte Kulturgefäße, die mit primären Kälbernierenzellkulturen bewachsen waren, wurden mit je 0,3 l serumfreiem Eagles minimum essential medium (Eagles MEM) mit Zusatz von 0,25 Gew.% Laktalbuminhydrolysat (LAH) beschickt, dem BHV 1 im Verhältnis 1:100 beigemischt war. 40 Stunden nach Infektion und Inkubation der Zellkulturen bei 37° C war der Höhepunkt der virusspezifischen Zellzerstörung erreicht, und die BHV 1-Kulturvirussuspension wurde geerntet, bei 2500 x g zentrifugiert sowie über einen Membranfilter von 0,65 µm Porenweite filtriert. Die insgeamt 20 l grob gereinigte Kulturvirussuspension wurden geteilt. Die eine Hälfte wurde über eine Ultrafiltermembran aus Zellulosenitrat mit einer Porenweite von 0,07 µm 10-fach konzentriert. Das 1 l umfassende Viruskonzentrat wurde nachfolgend mit 5 l PBS, pH 7,2, gewaschen. Die Waschflüssigkeit wurde dem Viruskonzentrat über das Ultrafilter wieder entzogen. Danach wurde das gewaschene Viruskonzentrat mit 100 g PEG, MG 6000, versetzt und 18 Stunden bei 5 bis 8° C auf einem Magnetrührer gerührt. Das entstandene Viruspräzipitat wurde in einer Kühlzentrifuge bei 2500 x g abzentrifugiert und in 0,5 l PBS, pH 7,2, gelöst. Das somit 20-fach konzentrierte und gereinigte BHV 1-Konzentrat wurde mit 0,25 ml Äthylenimin versetzt und 48 Stunden bei 26° C gerührt. Abschließend wurden die Reste des Äthylenimins mit 0,96 g Natriumthiosulfat abgebunden. Auf diese Weise wurde Testantigen I erhalten.

Die zweite Hälfte der grob gereinigten Kulturvirussuspension wurde über eine Ultrafiltermembran aus Polysulfon mit einer gegenüber dem erfindungsgemäßen Verfahren feineren Abscheidegrenze von Mol.-Gew. 100.000 ebenfalls 10-fach konzentriert und in der oben geschilderten Weise weiterbehandelt. Auf diese Weise wurde das Vergleichsantigen II erhalten.

Beide Antigene wurden vor ihrer Virusinaktivierung durch Virustitration auf ihren BHV 1-Gehalt überprüft. Der Virusgehalt des Testantigens I betrug pro ml 10⁹ʼ³⁶ Gewebekultur-infektiöse Dosis 50% (=GKID₅₀) der Virusgehalt des nicht nach dem erfindungsgemäßen Verfahren gereinigten Antigens II lag bei 10⁹ʼ⁴⁰ GKID₅₀/ml, d.h. im Virusgehalt war kein Unterschied festzustellen.

Beide BHV 1-Antigene wurden an BHV 1-durchseuchten, serologisch positiven Rindern durch intrakutane Injektion von 0,1 ml/Tier geprüft. Kriterium für ein positives Testresultat ist eine im Zeitraum von 2 bis 3 Tagen nachweisbare Hautdickenzunahme am Injektionsort um mehr als 2 mm.

Während mit dem nach dem erfindungsgemäßen Verfahren hergestellten BHV 1-Testantigen I von 270 seropositiven Tieren, die älter als 6 Monate waren, 266 (98,5 %) im Intrakutantest deutlich positiv reagierten, waren es von 84 seropositiven Tieren, die mit dem nicht erfindungsgemäß hergestellten Vergleichsantigen II geprüft worden waren, lediglich 67 (79,8 %), d.h. das nach dem erfindungsgemäßen Verfahren hergestellte BHV 1-Testantigen war zur Diagnosestellung bei seropositiven Tieren weitaus besser geeignet.

Von 345 über 6 Monate alten Rindern, die serologisch gegen BHV 1 negativ reagiert hatten, erwiesen sich 344 (99,7 %) Tiere nach Anwendung des erfindungsgemäß hergestellten Testantigens I ebenfalls negativ. Mit dem nicht erfindungsgemäß hergestellten Antigen II wurden 284 seronegative Rinder geprüft, von denen nur 271 (95,4 %) im Intrakutantest gleichfalls negativ reagierten, d.h. das nach dem erfindungsgemäßen Verfahren hergestellte Testantigen zeigte auch gegenüber BHV 1 seronegativen Tieren eine höhere Spezifität.

Bei Kälbern unter 6 Monaten, die über mütterliche, d.h. passiv über die Kolostralmilch erworbene BHV 1-Antikörper verfügten, war zwischen dem erfindungsgemäßen und dem nicht erfindungsgemäß hergestellten Antigen kein Spezifitätsunterschied erkennbar. Sowohl 30 mit dem nach dem erfindungsgemäßen als auch 14 mit dem nicht nach dem erfindungsgemäßen Verfahren hergestellten Testantigen untersuchte Kälber reagierten erwartungsgemäß negativ.

### Beispiel 2

Von 60 mit bovinen embryonalen Lungenzellkulturen bewachsenen, gerollten Kulturgefäßen wurde in der in Beispiel 1 geschilderten Weise BHV 1-Kulturvirussuspension gewonnen und durch Zentrifugation und Filtration von Zellresten befreit. Danach wurde die eine Hälfte mit einem Volumen von 10 l wiederum nach dem in Beispiel 1 geschilderten, erfindungsgemäßen Verfahren durch Ultrafiltration konzentriert, durch Waschen und Viruspräzipitation gereinigt und mit PBS in 5 Vol.% der Ausgangsmenge gelöst, während die andere Hälfte der Virussuspension nur durch eine Ultrafiltration 10-fach konzentriert, nicht aber mit PBS gewaschen und mit PEG 6000 präzipitiert, d.h. nicht gereinigt wurde. Nachfolgend wurden beide Antigene in der in Beispiel 1 geschilderten Weise mit Äthylenimin inaktiviert. Die Reste des Äthylenimins wurden wiederum mit Natriumthiosulfat abgebunden. Der vor der Inaktivierung ermittelte Virusgehalt des nach dem erfindungsgemäßen Verfahren hergestellten Antigens III lag bei 10^{8,9} GKID₅₀/ml und der des nicht erfindungsgemäß gereinigten Antigens IV lag bei 10^{8,5} GKID₅₀/ml.

Beide Antigene wurden wiederum BHV 1-durchseuchten, seropositiven Rindern intrakutan appliziert. Mit dem nach dem erfindungsgemäß hergestellten Antigen III wurden 74 Tiere untersucht, die ausnahmslos positiv reagierten, während mit dem zu Kontrollzwecken nicht nach dem erfindunggemäßen Verfahren hergestellten Antigen IV von 124 Tieren lediglich 62 (50%) eine als positiv zu wertende Hautdickenzunahme zeigten, d.h. allein das nach dem erfindungsgemäßen Verfahren hergestellte BHV 1-Testantigen verfügte über die zur BHV 1-Diagnosestellung erforderliche Spezifität.

## Patentansprüche

1. Verfahren zur Gewinnung eines Bovinen Herpes Virus Typ 1 (BHV-1) Antigens aus einer zellfrei filtrierten Suspension von BHV 1-Virus, worin die Virussuspension konzentriert und Verunreinigungen abgetrennt, das Viruskonzentrat mit einer Pufferlösung gewaschen, die Viren ausgefällt, das Viruspräzipitat in einer Pufferlösung suspendiert und die Viren inaktiviert werden, dadurch gekennzeichnet, daß Verunreinigungen abgetrennt werden, die ein Filter mit der Porenweite 0,06 - 0,08 µm passieren.

2. Verfahren nach Anspruch 1, dadurch gekennzeichnet, daß durch Ultrafiltration konzentriert wird.

3. Verfahren nach Anspruch 1, dadurch gekennzeichnet, daß mit einer isotonischen gepufferten Salzlösung gewaschen wird.

4. Verfahren nach Anspruch 1, dadurch gekennzeichnet, daß die Viren mit Polyäthylenglykol ausgefällt werden.

5. Verfahren nach Anspruch 1, dadurch gekennzeichnet, daß die Viren in einer isotonischen gepufferten Salzlösung suspendiert werden.

6. Verfahren nach Anspruch 1, dadurch gekennzeichnet, daß mit einem Zellulosenitratfilter mit einer Porenweite von 0,06 bis 0,08 µm, vorzugsweise von 0,07 µm ultrafiltriert, mit isotonischer phosphatgepufferter Salzlösung mit einem pH-Wert von 7,2 gewaschen und gelöst und mit Polyäthylenglykol mit einem Molekulargewicht von 6000 ausgefällt wird.

7. Diagnostisches Mittel enthaltend ein Antigen erhältlich gemäß Anspruch 1.

## Claims

1. A process for obtaining a bovine herpes virus type 1 (BHV 1) antigen from a suspension of BHV 1 virus filtered free of cells, in which the virus suspension is concentrated and contaminants are removed, the virus concentrate is washed with a buffer solution, the viruses are precipitated, the virus precipitate is suspended in a buffer solution, and the viruses are inactivated, which comprises contaminants which pass through a filter with a pore width of 0.06 - 0.08 µm being removed.

2. The process as claimed in claim 1, wherein concentration is by ultrafiltration.

3. The process as claimed in claim 1, wherein an isotonic buffered salt solution is used for washing.

4. The process as claimed in claim 1, wherein the viruses are precipitated with polyethylene glycol.

5. The process as claimed in claim 1, wherein the viruses are suspended in an isotonic buffered salt solution.

6. The process as claimed in claim 1, wherein a cellulose nitrate filter with a pore width of 0.06 to 0.08 µm, preferably of 0.07 µm, is used for ultrafiltration, isotonic phosphate-buffered salt solution with a pH of 7.2 is used for washing and dissolving, and polyethylene glycol with a molecular weight of 6000 is used for precipitation.

7. A diagnostic agent containing an antigen obtainable as claimed in claim 1.

## Revendications

1. Procédé pour produire un antigène du virus de type 1 de l'herpès bovin (BKV-1) à partir d'une suspension filtrée, dépourvue de cellules, de virus BVH-1, dans lequel on concentre la suspension de virus et on la débarrasse des impuretés, on lave le concentré de virus avec une solution tampon, on fait précipiter les virus, on met le précipité de virus en suspension dans une solution tampon et on inactive les virus, caractérisé en ce que l'on élimine les impuretés qui passent à travers un filtre dont la largeur de pores vaut de 0,06 à 0,08 µm.

2. Procédé selon la revendication 1, caractérisé en ce que l'on effectue la concentration par ultrafiltration.

3. Procédé selon la revendication 1, caractérisé en ce que l'on effectue le lavage avec une solution saline tamponnée isotonique.

4. Procédé selon la revendication 1, caractérisé en ce que l'on fait précipiter les virus avec du polyéthylène-glycol.

5. Procédé selon la revendication 1, caractérisé en ce que l'on met les virus en suspension dans une solution saline tamponnée isotonique.

6. Procédé selon la revendication 1, caractérisé en ce que l'on effectue l'ultrafiltration avec un filtre en nitrate de cellulose ayant une largeur de pores valant de 0,06 à 0,08 µm, de préférence, égale à 0,07 µm, on effectue le lavage et la dissolution avec une solution saline tamponnée au phosphate, isotonique, de pH 7,2 et on effectue la précipitation avec un polyéthylène-glycol dont la masse moléculaire est de 6000.

7. Agent de diagnostic contenant un antigène qu'il est possible d'obtenir conformément à la revendication 1.
